# EUROPEAN PATENT SPECIFICATION

(11) **EP 4 312 026 B1**
(45) Date of publication and mention of the grant of the patent: **29.04.2026**
(21) Application number: 23187290.4
(22) Date of filing: 24.07.2023
(51) Int. Cl.: G01N 33/00

(54) **ASPIRATION BASED MEASUREMENT AND ANALYSIS OF MULTIPOINT GASES**
AUF ASPIRATION BASIERENDE MESSUNG UND ANALYSE VON MEHRPUNKTGASEN
MESURE ET ANALYSE DE GAZ MULTIPOINTS BASÉES SUR L'ASPIRATION

(30) Priority: 29.07.2022 IN 202211043529
(43) Date of publication of application: 31.01.2024
(73) Proprietor: Honeywell International Inc., Charlotte, NC 28202 (US)
(72) Inventor: KOTHARI, Sanjay, Charlotte, 28202 (US); SUBRAMANIAN, Hariprasath, Charlotte, 28202 (US); PRABHU, Prashant, Charlotte, 28202 (US); KHURANA, Mohit, Charlotte, 28202 (US)
(74) Representative: Haseltine Lake Kempner LLP

(56) References cited:
- EP-A1- 3 163 299
- EP-A2- 0 607 984
- CN-A- 108 982 765
- CN-A- 112 129 898
- CN-A- 113 984 958
- US-A1- 2006 234 621
- US-A1- 2016 063 833
- US-A1- 2019 137 126
- US-A1- 2020 003 447

## Description

### TECHNOLOGICAL FIELD

Example embodiments of the present invention relate generally to aspiration based measurement and analysis of gases, and more particularly aspiration based measurement and analysis of multipoint gases and air quality, including from harsh environments.

### BACKGROUND

Many environments are difficult to measure and analyze gases in, including confined spaces and harsh environments. Confined spaces may be limited in physical size and present hazards to personnel that may enter the space. Many confined spaces, however, are also locations in which hazardous gases may be generated or are locations that contain equipment where gases detected may be indicative of a problem with or failure of equipment in the space. For example, batteries, such as lithium-ion batteries, may be used to store energy in cars, homes, business, and industrial plants. Batteries may be kept in such locations, and batteries may generate one or more gases during off gassing prior to thermal runaway. These gasses may be indicative of abuse the batteries have received and may be a warning of potential hazards to come, such as a fire hazard with thermal runaway. Similarly, residential, commercial, and industrial environments may also include spaces where gas measurement and analysis may be useful to identify and take action before an issue in that space becomes a problem, which may be due to materials kept in or near these environments or operations that occur in these areas. Moreover, often there are multiple locations in these environments to be monitored.

The detection of gases has conventionally been with single point gas monitors or with multipoint gas monitors that rely on diffusion type detection. As described herein, Applicant has identified a number of deficiencies, challenges, and problems associated with systems, apparatuses, methods, and computer program products for measurement and analysis of gases and air quality. Through applied effort, ingenuity, and innovation, many of these deficiencies, challenges, and problems have been solved by developing solutions that are included in embodiments of the present disclosure, some examples of which are described in detail herein.

Reference may be made to any of:
US 2006/234621 A1, which relates to a system for monitoring air quality conditions, comprising, a multi-point air monitoring system comprising, a plurality of sensors for collecting air quality data from a plurality of at least partially enclosed areas, one or more data processing units for processing one or more air quality parameters based on the collected air quality data, and one or more communication devices for communicating the data from the sensor to the processing unit, and a signal processing controller that generates one or more blended air quality parameter signals via the multi-point air monitoring system based at least in part on one or more of the processed air quality parameters representative of data from a plurality of the sensors;
EP 3 163 299 A1, which relates to a gas detection system comprising a sample gas inlet, a reference gas inlet and a gas modulation valve alternatingly connecting one of the sample gas inlet and the reference gas inlet to a gas sensor, wherein a selective transfer filter is located in the gas flow path connecting the gas modulation valve and the gas sensor; and
US 2020/003447 A1, which relates to an artificial intelligence (AI) device which includes a display, a communication unit configured to transmit indoor air quality information received from an air quality measurement device and meta information input through the display to an AI server, and a processor configured to receive an air quality analysis report generated based on outdoor air quality information, the indoor air quality information and the meta information from the AI server through the communication unit and display the received air quality analysis report on the display, wherein the air quality analysis report includes a first analysis report including a result of analyzing an indoor air quality condition during a measurement period of the air quality measurement device, and a second analysis report including an air quality type according to the indoor air quality condition and a solution for fine dust management according to the air quality type.

### BRIEF SUMMARY

Various embodiments described herein relate to a system and a method specifically for measurement and analysis of multipoint gases and air quality. The present invention is defined by the independent claims, to which reference should now be made. Specific embodiments are defined in the dependent claims.

In accordance with one aspect of the present disclosure, an aspiration based system is provided. The aspiration based system comprises a distribution subsystem including an inlet for each of a plurality of gas sources originating from batteries in multiple spaces, an aspiration subsystem coupled to the distribution subsystem, wherein the aspiration subsystem generates a vacuum; a gas analyzing unit; wherein, in operation, the aspiration based system is configured to: operate the distribution subsystem to collect, via the vacuum, a gas sample from each of the plurality of gas sources in a sequence via the associated inlet; measure, with the gas analyzing unit, the gas samples collected to generate one or more gas sample measurements; determine, based on an alarm threshold, an alarm state for one or more of the gas sample measurements; and generate an alarm based on the determination of the alarm state.

In some instances, the aspiration based system further comprises a gas conditioning subsystem, wherein the gas conditioning subsystem is connected to an output of the aspiration subsystem and an input to the gas analyzing unit; and wherein, in operation, the aspiration based system is further configured to: condition, prior to determining an alarm state, the gas samples collected. In some instances, the gas conditioning subsystem comprises a plurality of gas conditioners.

In some instances, the distribution subsystem comprises a plurality of valves.

In some instances, the aspiration subsystem comprises a plurality of aspiration pumps.

In some instances, the aspiration based system further comprises a purging subsystem.

In some instances, the aspiration based system further comprises a cooling subsystem.

In some instances, in operation, the aspiration based system is further configured to: generate an alert based on the determination of the alarm state; and transmit the alert to a computing device associated with a user.

The threshold is based on a model generated by a machine learning model.

The one or more of the plurality of gas sources are each associated with a plurality of battery cells.

In accordance with another aspect of the present disclosure, a method to measure and analyze multiple gas sources associated with a plurality of battery cells located in multiple spaces comprises: providing an aspiration based system comprising: a distribution subsystem including an inlet for each of the plurality of gas sources; an aspiration subsystem coupled to the distribution subsystem, wherein the aspiration based subsystem generates a vacuum; a gas analyzing unit; operating the distribution subsystem to collect, via the vacuum, a gas sample from each of the plurality of gas sources in a sequence via the associated inlet; measuring, with the gas analyzing unit, the gas samples collected to generate one or more gas sample measurements; determining, based on an alarm threshold, an alarm state for one or more of the gas sample measurements; and generating an alarm based on the determination of the alarm state.

In some instances, the aspiration based system further comprises a gas conditioning subsystem, wherein the gas conditioning subsystem is connected to an output of the aspiration subsystem and an input to the gas analyzing unit; and the method further comprises conditioning, prior to determining an alarm state, the gas samples collected.

In some instances, the method further comprises generating an alert based on the determination of the alarm state; and transmitting the alert to a computing device associated with a user.

The foregoing brief summary is provided merely for purposes of summarizing some example embodiments illustrating some aspects of the present disclosure. Accordingly, it will be appreciated that the above-described embodiments are merely examples and should not be construed to narrow the scope of the present disclosure in any way. It will be appreciated that the scope of the present disclosure encompasses many potential embodiments in addition to those summarized herein, some of which will be described in further detail below.

### BRIEF SUMMARY OF THE DRAWINGS

Having thus described certain example embodiments of the present disclosure in general terms, reference will now be made to the accompanying drawings, which are not necessarily drawn to scale, and wherein:
FIG. 1 illustrates an example system for aspiration based measurement and analysis of multipoint gases and air quality in accordance with one or more embodiments of the present invention;
FIG. 2 illustrates a further example system for aspiration based measurement and analysis of multipoint gases and air quality in accordance with one or more embodiments of the present invention;
FIG. 3 illustrates a further example system for aspiration based measurement and analysis of multipoint gases and air quality in accordance with one or more embodiments of the present invention;
FIG. 4 illustrates an example gas analyzing unit in accordance with one or more embodiments of the present invention;
FIG. 5 illustrates a block diagram of an example computing device configured to, in whole or in part, perform various operations described herein;
FIG. 6 illustrates a flowchart according to an example method for measurement and analysis of multipoint gases and air quality in accordance with one or more embodiments of the present disclosure;
FIG. 7 illustrates a flowchart according to an example method for operating a distribution subsystem in accordance with one or more embodiments of the present disclosure;
FIG. 8 illustrates a flowchart according to an example method for operating a gas conditioning subsystem in accordance with one or more embodiments of the present disclosure; and
FIG. 9 illustrates a flowchart according to an example method for measuring and analyzing gas samples in accordance with one or more embodiments of the present disclosure.

### DETAILED DESCRIPTION

Some embodiments of the present disclosure will now be described more fully herein with reference to the accompanying drawings, in which some, but not all, embodiments of the disclosure are illustrated. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

The use of the term "circuitry" as used herein with respect to components of an apparatus should be understood to include particular hardware configured to perform the functions associated with the particular circuitry as described herein. The term "circuitry" should be understood broadly to include hardware and, in some embodiments, software for configuring the hardware. For example, in some embodiments, "circuitry" may include processing circuitry, storage media, network interfaces, input/output devices, and the like. In some embodiments, other elements may provide or supplement the functionality of particular circuitry.

The term "electronically coupled," "electronically coupling," "electronically couple," "in communication with," or "in electronic communication with," in the present disclosure refers to two or more elements, modules, or components being connected through wired means and/or wireless means, such that signals, electrical voltage/current, data and/or information may be transmitted to and/or received from these elements or components.

As used herein, the term "data object" refers to electronically managed data capable of being collectively transmitted, received, and/or stored. A data object may be defined by one or more associated data structures, which may include at least a data structure identifier that uniquely identifies a data structure represented by the data object and/or the data object itself. For example, a data object may refer to a collection of data and instructions that represent data of a computing device and/or data of a plurality of computing devices. For example, a data object may comprise one or more data structures defined by data properties. In some embodiments, a data object may be associated with any of a number of related data objects, such as a computing device data object. For example, a data object utilized by a computing device or a device information system may comprise a data object identifier, a data structure identifier, a file(s) created and stored within the device information system, metadata associated with one or more data elements, structures, objects, and/or packets, and the like, or any combination thereof.

### Exemplary System

Various embodiments of the present invention are directed to an improved system and method for aspiration based gas measurement and analysis, and more particularly for measurement and analysis of gases originating from battery cells located in multiple spaces.

Conventional systems include single point measurement solutions that locate a gas monitor at a single location or locate gas monitors at multiple locations with standalone monitors at each location that may separately measure and analyze gases. The use of separate gas monitors may also include calibration problems or inconsistency between measurements for variations in the different gas monitors. Further, conventional gas monitors utilize dispersion where a gas is allowed to disperse into an environment and a gas monitor will wait for the gas to reach the gas monitor before detecting a gas. Dispersion monitors are slower to detect gases as a gas concentration may not disperse in the direction of the gas monitor or do so only in low quantities. Such conventional systems would also not leverage measurement and analysis of gas samples from multiple points consistently, including any interactions between the multiple points as well as historical data. Additionally, the use of separate gas monitors fails to allow for measurements of multiple gas monitors to be used to draw inferences or assist in the analysis of other locations.

The present invention uses an aspiration based multipoint system for measurement and analysis of gases and air quality ("system" or "system 100"). The system uses current and historical measurements from multiple points to analyze gas samples. The analysis of the gas samples not only confirms that the system is working but also may be used to generate alarms and/or alerts or be used to, directly or indirectly, control equipment in or around a point from which the gas sample is taken from.

The system is an aspiration system that utilizes a vacuum created by one or more aspiration pumps to generate gas samples from multiple points referred to herein as gas sources. The system intakes gas samples from multiple gas sources. In some embodiments, there may be multiple sources in one space (e.g., a room), which may be dependent on the equipment in the space. For example, each point for a gas source may be a separate battery, battery cell, or space containing a plurality of batteries (e.g., a battery rack with multiple batteries in a battery room). The vacuum created by an aspiration subsystem in the system causes air from the gas source to be drawn into the system. The amount of air drawn by the vacuum may be controlled by the use of one or more valves in a distribution subsystem along with controlling the vacuum to control how gas samples are collected and distributed from different gas sources to a gas analyzer unit. By controlling the collection and distribution of gas samples, the system may measure and analyze the gas samples from multiple gas sources in a controlled manner that utilizes current data and historical data to determine if the gas sample may be associated with an alarm state.

The system is used to monitor different spaces that may contain or be associated with one or more gas sources. Such spaces are associated with energy storage systems, for batteries. Some of these spaces may contain hazardous environments or inflammable gases. The spaces may be located near one another (e.g., battery compartments in an electric vehicle) or may be located apart.

The spaces may contain one or more materials or equipment that may generate one or more gasses. In various embodiments with batteries, batteries may generate off gasses when they are abused. Battery abuse may include mechanical shock (e.g., crush, penetration), overheat, overcharge, electrolyte leakage, drawing more current than rated (e.g., short circuit), etc. A battery with electrolytes may, with such abuse, have the electrolytes become pressurized due to the abuse and, thus, release off gasses. This may include electrolyte coming out of the battery as a vapor. Such off gassing may be indicative of abuse having occurred and may be an indicator of future damage. Indeed, off gassing commonly occurs before and during thermal runaway then a battery may generate heat that will cause damage to the battery, including generation of smoke and/or the catching of fire due to the thermal increase and destruction of the components of the battery. By detecting the off gassing early any further damage, as well as any generation of smoke or starting of a fire, may be prevented by generating alarms and or controlling the taking of one or more corrective actions.

The spaces to be monitored may be stationary or, alternatively, they may be mobile. For example, data centers, wind farms, solar farms, power plants, industrial facilities, and the like contain stationary spaces that may be monitored as gas sources. In contrast, mobile transportations (e.g., electric vehicles, ships, planes, and the like) may contain batteries or other spaces that may be monitored.

The gas sources will be sampled in a sequential order as described herein to measure and analyze the accumulation of gases.

The system may measure gas samples and analyze the gas samples for conditions that may lead to hazardous levels of gases and/or combustible levels of gases.

In various embodiments, the spaces to be monitored may be confined spaces where access is restricted. Confined spaces may include environments that may be hazardous due to the size, shape, or location. For example, a manhole or tank may collect gases and have limited, if any, air exchanges. The confined space, due to its collection of gas, may lead to dangerous situations, including an explosive environment or an environment where there is not sufficient air for a person to breathe. These spaces may be difficult to monitor, including having limited space in which to place a gas monitor. Moreover, aside from space for a gas monitor, there may be insufficient space for installation or maintenance, which may be due to the hazardous conditions of the space. The placement of gas monitors may also be limited by the gases in the environment as the operation of a gas monitor and accompanying equipment may, for example, ignite gases in an explosive environment.

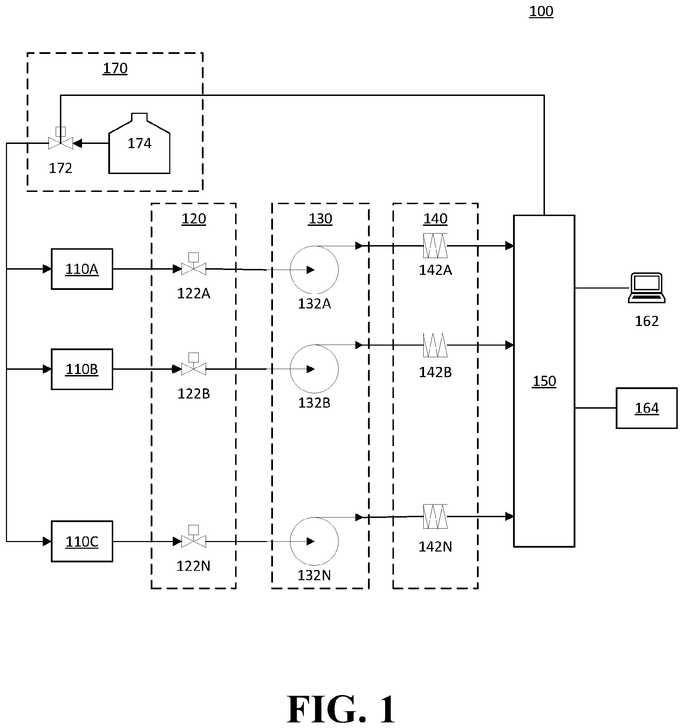

FIG. 1 illustrates an example system 100 for aspiration based measurement and analysis of multipoint gases and air quality in accordance with one or more embodiments of the present invention. As illustrated, system 100 includes a plurality of gas sources 110, such as a first gas source 110A, a second gas source 110B, and an N^{th} gas source 110N. While FIG. 1 illustrates 3 gas sources, any number of gas sources may be monitored. For example, in various embodiments, there may be sixty-four (64) gas sources that are monitored. Each of the gas sources 110 may be monitored for the same gas or each may be monitored for different gases or combinations of gases.

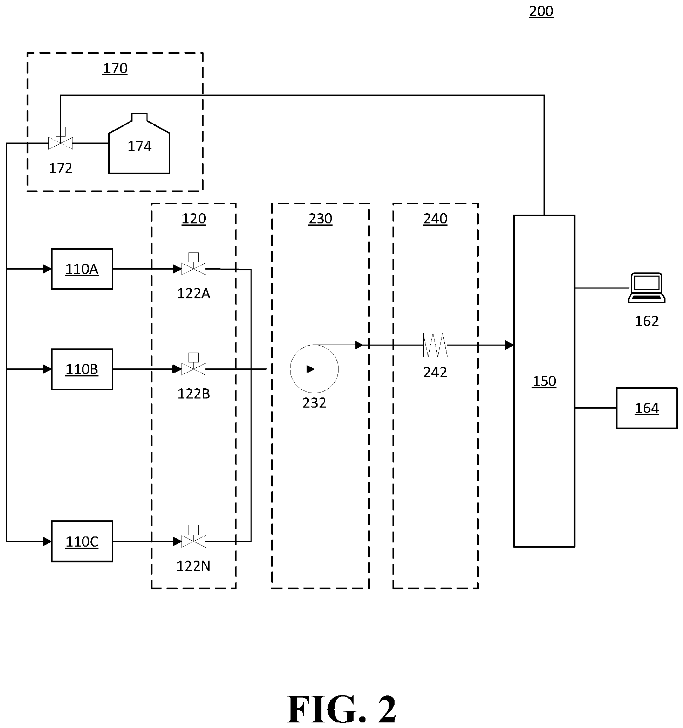

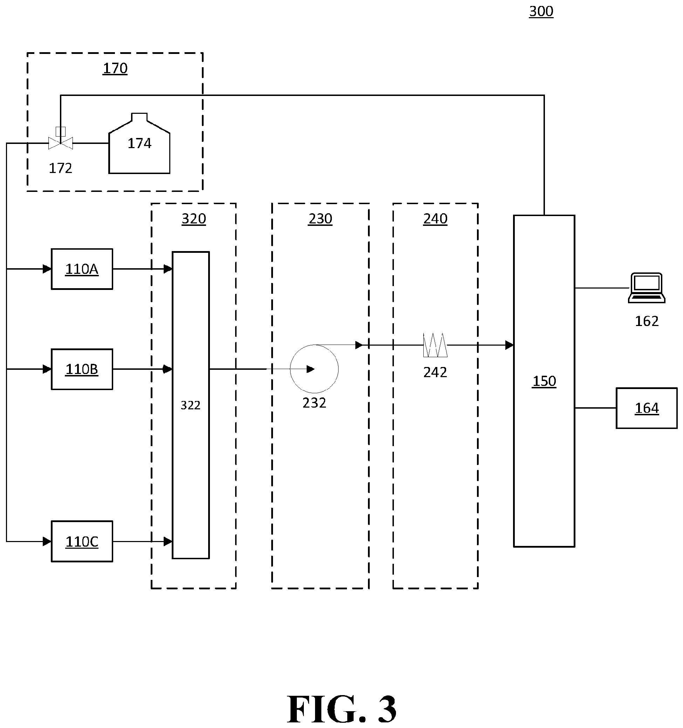

From each gas source 110 to the gas analyzing unit 150 there may be a plurality of channels, with each channel utilizing one or more subsystems. FIG. 1 illustrates 3 channels. The first channel including the connections between gas source 110A and gas analyzing unit 150; the second channel including the connections and connected equipment between gas source 110B and gas analyzing unit 150; and the third channel including the connections and connected equipment between gas source 110N and gas analyzing unit 150. Similarly, FIGs. 2 and 3 also illustrate 3 channels. The channels illustrated in FIG. 1 between the gas sources 110 and the gas analyzing unit 150 includes piping to transport a gas sample from a gas source 100 to the gas analyzing unit 150.

Multiple subsystems may be included in the system 100. FIG. 1 illustrates four subsystems: distribution subsystem 120, aspiration subsystem 130, gas conditioning subsystem 140, and purging subsystem 170. As illustrated in FIGS. 1-3, these subsystems may be configured in more than one manner.

The distribution subsystem 120 of FIG. 1 illustrates a distribution system 120 with three valves 122A, 122B, 122N with each valve 122 being associated with one of the three illustrated gas sources 110A, 110B, 100N. In the configuration illustrated in FIG. 1, each of the gas sources 110 is associated with a single valve 122. A gas sample of a gas source 100 may be generated by opening a valve 122 to allow for gas from the environment of a gas source 110 to be collected by an inlet of a piping connection that connects a gas source 110 with a valve 122. Thus while the distribution subsystem 120 of FIG. 1 is illustrated as a separate block from gas source 110, the location of an inlet or valve of a distribution subsystem 120 may be location at or in a gas source 110. The gas sample is collected due to suction of the aspiration based system 100, which draws in gas samples from an environment of a gas source 110. Closing the valve 122 associated with a gas source 110 shuts off the environment from the gas source 110 and ends the amount of gas that will be in a gas sample to be measured and analyzed. In the configuration of FIG. 1, with each gas source 110 being associated with its own channel valve 122, the distribution system 120 will include multiple output ports that are connected to inputs of the aspiration subsystem 130.

In various embodiments, the distribution subsystem may include one or more additional equipment (not depicted) to control or maintain a valve 122 (or similar equipment) or distribute a gas sample from a gas source 100 to an aspiration subsystem 130. For example, a distribution subsystem may include more than one valve 122 per channel, which may provide further isolation from a gas source 110 depending on one or more environments a gas sample may be transported through. For example, there may be additional values to provide for routing, isolation, or exhaust of gas samples. Additionally, a distribution may include one or more electrical control panels or boxes to control or provide signals to or from one or more pieces of equipment of the distribution subsystem 120.

The aspiration subsystem 130 of FIG. 1 illustrates a plurality of aspiration pumps 132A, 132B, 132N, and each valve 122 may be associated with its own aspiration pump 132. An aspiration pump 132 will generate a vacuum that draws in a gas sample from a gas source 110 via a distribution subsystem 120. As illustrated in FIG. 1, each channel may be having its own aspiration pump 132 such that a gas source 110 may be associated with one value 122A and one aspiration pump 132A.

The gas conditioning subsystem 140 of FIG. 1 illustrates a plurality of gas conditioners 142A, 142B, 142N with each valve 122 and aspiration pump 132A being associated with its own gas conditioner 142A. A gas conditioner 142 may condition a gas sample by changing one or more characteristics of the gas sample. In various embodiments, such characteristics include, but are not limited to, temperature, pressure, and/or humidity. In various embodiments, temperature may be changed by cooling or heating a gas sample. In various embodiments, humidity may be changed by causing water vapor to condense or by introducing gas vapor. In various embodiments, a gas conditioner 142 may be a condenser and/or a portion or all of an HVAC subsystem. The gas conditioning subsystem 140 may also include one or more temperature measurement device and/or humidity measurement devices that measure a temperature and/or humidity of the gas sample before and after gas conditioning. Such measurement devices may be used to determine when a gas has been sufficiently conditioned to be passed from the gas conditioning subsystem 140 to the gas analyzer unit 150.

The gas conditioners 142 may condition a gas sample to have specific characteristics that are to match or accommodate the requirements of a gas monitor. The system 100, in the gas analyzer unit 150, may contain one or more gas monitors. The gas monitors may include sensitive sensors that require a gas sample to, for example, be within a temperature range and/or humidity range. In various embodiments a gas sample may be taken from a gas source at 75C but a gas sensor (e.g., a non-dispersive infrared sensor (NDIR sensor)) may have an operating range requirement for gas samples with a maximum of 50C. In such embodiments, the gas conditioner may cool the gas sample to 50C or below so that the gas sample is within the operating range of the gas monitor. In various embodiments, the system 100 may provide a gas conditioner 142 a signal to control the gas conditioner 142 based on a requirement of a gas monitor. The requirements may vary based on, for example, the gas monitor to be used or a current status or state of a gas monitor.

The gas analyzing unit 150 may receive a gas sample, such as via a channel from a gas source 110, and measure and/or analyze the gas sample. The gas analyzing unit 150 may include one or more gas monitors (not depicted) that may measure and analyze one or more gases in the gas sample. In various embodiments, gas samples are measured and analyzed for carbon dioxide (CO2), carbon monoxide (CO), volatile organize compounds (VOCs) (e.g., H2, H2S, O2, hydrofluorocarbons, and the like). Additionally, or alternatively, gas samples are monitored for air quality, including temperature, pressure, humidity, particulate count, and the like, which may be used to determine a quality of the air in a gas sample. Measurement and analysis may be with one or more gas monitors, which may depend on how many gases on gas monitor may be able to measure and analyze. In various embodiments, distinct gas monitors may be used for each gas, particularly if gas monitors may require high degrees of sensitivity that may not be available in one gas monitor that may measure and analyze multiple gases.

The gas analyzing unit 150 may include a computing device as described herein. The computing device may be configured to perform one of more of the operations described herein. The computing device may also be configured to receive and transmit signals to one or more of the subsystems and/or one or more control panels 164 or control systems of equipment associated with a gas source 110.

In accordance with the invention, the gas analyzing unit 150 communicates with a battery control panel 164 to take one or more measurements of the batteries and may control, for example, the charging, discharging, and monitoring of the batteries. In various embodiments, the gas analyzing unit 150 may communicate with environment control equipment associated with the gas sources, such as an HVAC system, which may operate cool equipment (e.g., a battery) associated with a gas source 110. In various embodiments, such cooling equipment may be configured to provide cooling, such as forced cooling, to equipment associated with a gas source.

The system 100 may be in communicative connection with a computing device 162. In various embodiments the computing device 162 may be a local or remote computer that may be connected to the system 100. The computing device 162 may allow for a user to connect to and/or receive data, alarms, and/or alerts from the system 100. In various embodiments, when an alert is generated by the system 100, the alert may be transmitted to a user at computing device 162. While FIG. 1 illustrates one computing device 162, it will be appreciated that the system 100 may be connected to one or more computing devices 162, and such connections may be over a network, which may be wired or wireless and may be private networks or public networks (e.g., the internet). In various embodiments, the computing device 162 may include a display that may display one or more signals or data objects received from the system 100. In various embodiments, the one or more signals or data objects may be rendered in a visualization or dashboard, and the system 100 may generate a data object to be rendered. Additionally, or alternatively, the system 100 may cause the visualization or dashboard to be rendered on a display, which may be based on an alarm signal or alert generated by the system 100.

The system 100 is in communicative connection with a control panel 164.

In accordance with the invention the control panel (164) is a control panel associated with batteries stored in locations that are being monitored as gas sources 110. This battery control panel provides signals and/or data objects to the system 100 and to the gas analyzing unit 150. Such signals and/or data objects are used in the analysis of gas samples. The system 100 receives one or more signals and or data objects, which include battery data, such as battery cell voltage for each battery cell, battery status (e.g., charging, discharging, on float, etc.), charging time, discharging time, temperature, and the like.

The purging subsystem 170 of FIG. 1 illustrates a valve 172 and a reservoir 174. The reservoir 174 may contain a purging substance, such as an inert gas (e.g., nitrogen, argon, etc.), that may be used to purge an environment associated with a gas source 110 of air or suppress a fire occurring or from occurring at a gas source 110. The purging substance may, for example, reduce the probability of a fire or thermal increase in the environment of the gas source 110. A signal from the gas analyzer unit 150 may be provided to the purging subsystem to cause valve 172 to open and the purging substance of reservoir 174 to be provided to each of the gas sources 110.

The purging subsystem 170 may include a reservoir 174 of nitrogen gas. When the system 100 may determine an alarm state associated with an indication of thermal runaway based one or more gas samples, the system 100 may generate an alarm signal and transmit it to the purging subsystem 170 to cause a valve 172 to open and the nitrogen be provided to each of the gas sources monitored. In various embodiments, the reservoir 174 may contain a substance that may be under pressure so that when valve 172 is opened the contents may be distributed by the purging subsystem to the gas sources 110.

In various embodiments, if the gas source 100 is in a space where personnel may be located, such as a clean room, and an alarm may be generated so that the personnel are alerted to evacuate the space before the purging subsystem 170 would provide or fill the space with a purging substance. Also, the purging subsystem 170 may be on a delayed timer from receiving an alarm to allow for personnel to exit the clean room. After the delayed timer expires, the doors will be closed and a purging system may cause, for example, an inert gas or a fire suppression system to fill the clean room. The substance in the reservoir 174 may be based on the specific manufacturing performed in the clean room and/or the types of hazardous gases used in the manufacturing process. Thus, the purging substance may be based on one or more materials in a space associated with a gas source 110. For example, if a space contained an inflammable material, then the purging substance may be a substance that prevents the material from combusting.

In various embodiments, the system 100 may be configured to control one or more cooling subsystems (not depicted) associated with one or more of the gas sources 110. For example, a space associated with a gas source may include one or more cooling sources, such as air conditioning, fans, louvers, etc. These cooling sources may be controlled with one or more signals generated by and transmitted from the system 100 to cool the equipment or material in the space associated with a gas source 110. For example, in embodiments where an increase in temperature may cause greater chemical reactions, the cooling may reduce the reaction's speed or prevent a reaction from progressing, which may also create time for a reaction to be addressed. Additionally, forced cooling may cause one or more air changes that may expel gas build up that may have occurred in a space. An air change may also allow for personnel to enter a space, with or without personal protection equipment, where prior to the air change gases in the space may have created a hazardous environment.

FIG. 2 illustrates a further example system 200 for aspiration based measurement and analysis of multipoint gases and air quality in accordance with one or more embodiments of the present invention. The system 200 illustrated in FIG. 2 is similar to the configuration of system 100 of FIG. 1 except that the aspiration subsystem 230 and gas conditioner subsystem 240 have a different configuration. In FIG. 2, the aspiration system 230 includes one aspiration pump 232. The aspiration pump 232 includes one input. The output of each of the values 122 of distribution subsystem 120 are connected so that a gas sample from each of the valves 122 may be input into the aspiration pump 232. The system 200 may control each of the valves 122 so that only one gas sample from one gas source 110 is generated at a time. For example valve 122A may be opened at a first time and closed at a second time to generate a first gas sample from gas source 110A that is transferred to the aspiration pump 232 due to the vacuum created by the aspiration pump 232. Subsequently, after this first gas sample has been transferred, valve 122B may be opened at a third time and closed at a fourth time to generate a second gas sample from gas source 110B that is transferred to the aspiration pump 232. The output of each of the valves 122 may be connected as only one gas sample will be collected at a time, which may be controlled by the system 200, such as with a computing device of the gas analyzer unit 150. The signals may be transmitted in the system 200 to control the valves 122 in a known manner to generate gas samples from each of the gas sources 110.

FIG. 3 illustrates a further example system 300 for aspiration based measurement and analysis of multipoint gases and air quality in accordance with one or more embodiments of the present invention. The system 300 illustrated in FIG. 3 is similar to the configuration of system 200 of FIG. 2 except that the distribution subsystem 320 has a different configuration. As illustrated in FIG. 3, distribution subsystem 320 includes a many-to-one valve 322, which may have multiple input ports and one output port. As illustrated in FIG. 3, the output of valve 322 may be connected to the input of aspiration pump 232. The system 300 may control the valves 322 so that only one gas sample from one gas source is generated at a time. For example valve 322 may be controlled such that an input port is opened to collect a gas sample from a first gas source 110A and then closed before a gas sample is collected from a second gas source 110B. As gas samples are collected they are transferred to the aspiration pump 232 due to the vacuum created by the aspiration pump 232. The system 300 may control valve 322 in a known manner to generate gas samples from each of the gas sources 110.

In various embodiments, the distribution subsystem 120 may be operated to collect gas samples from more than one source 110 at a time. For example, multiple gas sources 110 may be for one space, and gas samples from each inlet in the space may be used.

As is readily appreciated, variations to the illustrated configurations of system 100 are within the scope of the present disclosure. In various embodiments, a system 100 may include a plurality of valves 122 connected to a plurality of aspiration pumps 132 and/or a plurality of gas conditioners 142. The aspiration pumps 132 may be different, such as having different vacuum sizes, and the gas conditioners may be different, such as conditioning for different characteristics or for different gases. The system 100 may transfer a gas sample based on aspiration of various combinations of aspiration pumps 132 and/or gas conditioners 142.

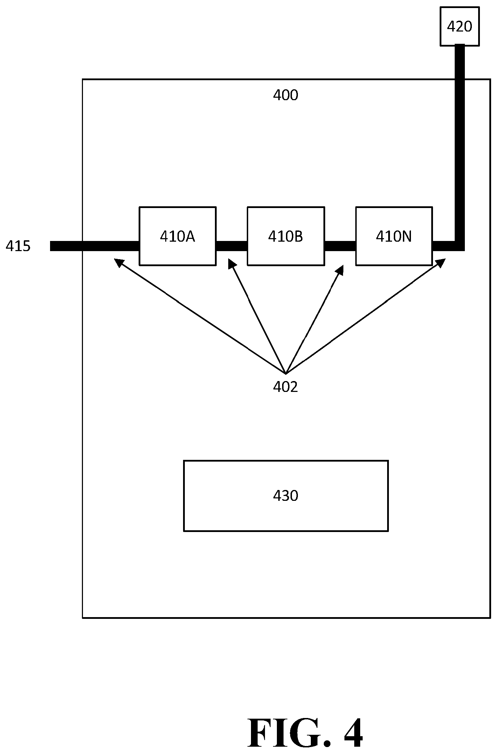

FIG. 4 illustrates an example gas analyzing unit 150 in accordance with one or more embodiments of the present invention. The gas analyzing unit 150 illustrated in FIG. 4 may include an enclosure 400 that may include piping 402 that may connect one or more gas monitors 410 (e.g., 410A, 410B, ... 410N) as well as an exhaust 420. The gas analyzing unit 150 may also include a computing device 430 that may be electrically coupled with the one or more gas monitors 410. A gas monitor 410 may be configured to measure one or more characteristics of the gas sample as well as, based on such measurements, detect the identity and/or concentration of one or more gases in the gas sample. The gas monitors 410N may generate one or more data signals or data objects associated with the measurements and transmit these data objects to the computing device 430 of the gas analyzing unit 150. Additionally, or alternatively, the gas monitors 410 may generate one or more signals (e.g., analog signals or digital signals) that may be transmitted to the computing device 430. The computing device 430 may be configured to receive the data object(s) and/or signals from the gas monitors 410 and analyze the gas sample to determine an alarm state and/or generate an alarm and/or an alert.

The gas monitors 410 may include high quality, high accuracy sensors. Such sensors may be located in one gas monitor 410 or may be located in more than one gas monitor 410. In various embodiments, a gas monitor 410 may include one or more sensors including, but are not limited to, non-dispersive infrared sensor (NDIR sensor), electrochemical sensor, photoionization detector (PID), and catalytic breed sensor.

In various embodiments, a computing device 430 may be integrated into a gas monitor 410. In various embodiments with a plurality of gas monitors 410, the gas monitors 410 may be electrically coupled to communicate with each other to execute one or more operations as described herein.

In various embodiments, the gas analyzer unit 150 may include one or more inlets 415 which may connect the gas analyzing unit 150 to a subsystem of the system 100, such as gas conditioning subsystem 140 or an aspiration subsystem 130. While FIG. 4 is illustrated with one inlet 415, there may be multiple inlets 415 that may be inputs into the gas analyzing unit 150 to allow for the receiving of one or more gas samples. In various embodiments, each of the inlets 415 may be connected to its own gas monitor 410, which may be connected to exhaust 420. Alternatively, each of the inlets 415 may be connected, such as with a junction having a single output, to a gas monitor 410, which may be the first in a series or combination of gas monitors 410.

In various embodiments, a gas analyzing unit 150 may contain one gas monitor 410. Alternatively, and as illustrated in FIG. 4, a gas analyzing unit 150 may contain one or more gas monitors 410 that are connected in series before being connected to an exhaust 420. In series connections, a gas sample may be received by the gas analyzing unit 150 and passed to a first gas monitor 410A that may detect the gas sample for one or more gases. The gas sample may then be passed to a second gas monitor 410B that may detect for one or more gases, some of which may be different gases than the first gas monitor 410A. The gas sample may be passed to additional gas monitors (e.g., 410N) until the gas analyzing unit 150 has finished measuring the gas sample with gas monitors 410.

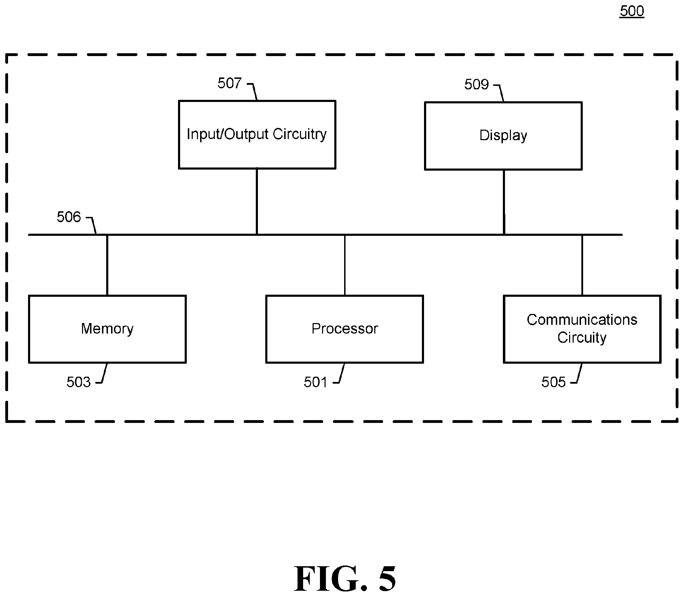

FIG. 5 illustrates a block diagram of an example computing device configured to, in whole or in part, perform various operations described herein. The gas analyzing unit 150 may include one or more computing devices, such as the apparatus 500 shown in FIG. 5. The apparatus 500 may include a processor 501, a memory 503, communications circuitry 505, input/output circuitry 507, and/or a display 509 that are in electronic communication with one another via a bus 506. The apparatus 500 may be configured to execute the operations described herein, which may include executing an operation in conjunction with one or more other portions, components, or subsystems of a system 100. Although the components are described with respect to functional limitations, it should be understood that the particular implementations necessarily include the use of particular hardware. It should also be understood that certain of the components described herein may include similar or common hardware. For example, two sets of circuitries may both leverage use of the same processor, network interface, storage medium, or the like to perform their associated functions, such that duplicate hardware is not required for each set of circuitries.

In some embodiments, the processor 501 may be embodied in a number of different ways and may, for example, include one or more processing devices configured to perform independently. Additionally, or alternatively, the processor 501 may include one or more processors configured in tandem via a bus to enable independent execution of instructions, pipelining, and/or multithreading.

For example, the processor 501 may be embodied as one or more complex programmable logic devices (CPLDs), microprocessors, multi-core processors, co-processing entities, application-specific instruction-set processors (ASIPs), and/or controllers. Further, the processor 501 may be embodied as one or more other processing devices or circuitry. The term circuitry may refer to an entirely hardware embodiment or a combination of hardware and computer program products. Thus, the processor 501 may be embodied as integrated circuits, application specific integrated circuits (ASICs), field programmable gate arrays (FPGAs), programmable logic arrays (PLAs), hardware accelerators, another circuitry, and/or the like. As will therefore be understood, the processor 501 may be configured for a particular use or configured to execute instructions stored in volatile or non-volatile media or otherwise accessible to the processor 501. As such, whether configured by hardware or computer program products, or by a combination thereof, the processor 501 may be capable of performing steps or operations according to embodiments of the present disclosure when configured accordingly.

In some embodiments, the memory 503 may be non-transitory and may include, for example, one or more volatile and/or non-volatile memories. In other words, for example, the memory 503 may be an electronic storage device (e.g., a computer readable storage medium). The memory 503 may be configured to store information, data, content, applications, instructions, or the like, for enabling the apparatus 500 to carry out various functions in accordance with example embodiments of the present disclosure. In this regard, the memory 503 may be preconfigured to include computer-coded instructions (e.g., computer program code), and/or dynamically be configured to store such computer-coded instructions for execution by the processor 501.

The processor 501 may be configured to execute instructions stored in the memory 503 or otherwise accessible to the processor. Alternatively, or additionally, the processor 501 may be configured to execute hard-coded functionality. As such, whether configured by hardware or software methods, or by a combination thereof, the processor may represent an entity (e.g., physically embodied in circuitry) capable of performing operations according to an embodiment of the present disclosure while configured accordingly. Alternatively, as another example, when the processor 501 is embodied as an executor of software instructions, the instructions may specifically configure the processor to perform the algorithms and/or operations described herein when the instructions are executed.

In some embodiments, the apparatus 500 may include the input/output circuitry 507 that may, in turn, be in communication with the processor 501 to provide output to the user and, in some embodiments, to receive an indication of a user input. The input/output circuitry 307 may comprise an interface, a mobile application, a kiosk, or the like. In some embodiments, the input/output circuitry 507 may also include a keyboard, a mouse, a joystick, a touch screen, touch areas, soft keys, a microphone, a speaker, or other input/output mechanisms. The processor and/or user interface circuitry comprising the processor may be configured to control one or more functions of one or more user interface elements through computer program instructions (e.g., software and/or firmware) stored on a memory accessible to the processor (e.g., memory 503).

The communications circuitry 505 may be any means such as a device or circuitry embodied in either hardware or a combination of hardware and software that is configured to receive and/or transmit data from/to a network and/or any other device, circuitry, or module in communication with the apparatus 500. In this regard, the communications circuitry 305 may include, for example, a network interface for enabling communications with a wired or wireless communication network and/or in accordance with a variety of networking protocols described herein. For example, the communications circuitry 505 may include one or more network interface cards, antennae, buses, switches, routers, modems, and supporting hardware and/or software, or any other device suitable for enabling communications via a network. Additionally, or alternatively, the communication interface may include the circuitry for interacting with the antenna(s) to cause transmission of signals via the antenna(s) or to handle receipt of signals received via the antenna(s).

In some embodiments, the apparatus 500 may include a display 509 that may, in turn, be in communication with the processor 501 to display user interfaces (such as, but not limited to, display of indicia or decoded indicia). In various examples of the present disclosure, the display 509 may include a liquid crystal display (LCD), a light-emitting diode (LED) display, a plasma (PDP) display, a quantum dot (QLED) display, and/or the like.

In system 100, multiple racks with batteries located in multiple spaces are monitored for gases. It is readily appreciate that a "battery" may be a collection of battery cells connected in a configuration to provide power with a specific voltage and/or current to a load. Types of batteries may include, but are not limited to, lithium ion batteries, sodium sulfur batteries, zinc bromide batteries, lead acid batteries, and the like. As the battery cells are physically distinct, abuse may occur to one or more battery cells while not occurring to others. The battery, or battery strings, with its collection of battery cells, may occupy different physical spaces which may be separated by one or more boundaries that make a plurality of spaces to be monitored. Each of the plurality of spaces may make up a gas source 110, or one space may be monitored with multiple gas sources 110, which may allow for the monitoring of groupings of spaces.

For example, a first battery rack comprised of multiple battery cells is located in a first space, and a second battery rack comprised of multiple battery cells is located in a second space. These may be, for example, battery rooms with the battery cells sitting in their battery racks. These which may also be confined spaces. Alternatively, or additionally, batteries may be stored in panels, cabinets, equipment, or in separate buildings to position the batteries close to where their power would be delivered. The system 100 is configured to monitor the first space as a first gas source 110A and the second space as a second gas source 110B. If one or more of the battery cells is abused, the battery cell may start releasing off gases, which may be indicative of a potential thermal runaway event.

The system 100 of FIG. 1 is configured to monitor the gas sources 110 by taking a first gas sample from a first gas source 110A and then, subsequently, a second gas sample from a second gas source 110B. The system 100 may iterate taking gas samples between the first gas source 110A and second gas source 110B. A gas sample may be taken by operating a valve 122A, 122B associated with the gas source 110A, 110B for a predetermined period of time to collect via suction a known quantity of air for a gas sample. After the predetermined period of time, the valve 122A, 122B may close. The gas sample is received at the respective aspiration pump 132A, 132B before being passed to a gas conditioner 142A, 142B. The gas sample from the battery may be at 75C and the gas conditioner 142A, 142B may condition the gas sample by lowering the temperature via a condenser below 50C. The gas condenser may pass the gas sample to the gas analyzing unit 150. The gas analyzing unit 150 may receive the gas sample, such as by operation of one or more valves that may distribute the gas sample to one or more gas monitors 410. The gas monitors 410 measure an abnormally high concentration of a gas, which may be above a threshold. In accordance with the invention, the threshold has been determined by the gas analyzer unit 150 based on a calibration and prior historical measurement data and data from a battery control system 164.

The system 100 controls the sampling of the gas sources 110 and, thus, the gas sample that generated an alarm state is from a known gas source 110. The instant gas sample is analyzed in view of measurements from one or more historical gas samples, stored in the memory of the system 100, such as in memory 503. The system 100 may determine a change in gas concentration over time, which may be indicative of, for example, thermal runaway in batteries. Additionally, the system 100 may receive the temperature of the gas sample, such as from a temperature measurement device at the gas source 110 as well as a temperature measurement device at a gas conditioner 142. The temperature(s) of the gas samples may also allow for the determination of an increase in temperature over time, which may be used in the determination of an alarm state associated with thermal runaway. In various embodiments, a control panel 164 may provide environmental data, such as an ambient temperature. The system 100 may use the ambient temperature in view of the gas sample data to determine if the temperature(s) of the gas samples over time rose more than a rise in ambient temperature, which may further indicate thermal runaway. On determination of an alarm sate associated with an indication of thermal runaway, the system 100 may generate an alarm and alert as described herein. The alarm may include an alarm signal that may cause a purging system 170 to purge the gas source 110 with an inert gas. The alert may include an alert to a computing device 162 that causes a rendering of an alert to an operator, which may be with a visualization or a dashboard.

The system 100 controls the sampling of the gas sources 110 and, thus, the gas sample that generated an alarm state is from a known gas source 110. The instant gas sample may be analyzed in view of measurements from one or more historical gas samples, which may be stored in the memory 503 of the system 100. The system 100 may determine a change in gas concentration over time, which may be indicative of, for example, thermal runaway in batteries. Additionally, the system 100 may receive the temperature of the gas sample, such as from a temperature measurement device at the gas source 110 as well as a temperature measurement device at a gas conditioner 142. The temperature(s) of the gas samples may also allow for the determination of an increase in temperature over time, which may be used in the determination of thermal runaway. In various embodiments, a control panel 164 may provide environmental data, such as an ambient temperature. The system 100 may use the ambient temperature in view of the gas sample data to determine if the temperature(s) of the gas samples over time rose more than a rise in ambient temperature, which may indicate thermal runaway. On determination of an indication of thermal runaway, the system 100 may determine there is an alarm state and generate an alarm and alert as described herein. The alarm may include an alarm signal that may cause a purging system 170 to purge the gas source 110 with an inert gas. The alert may include an alert to a computing device 162 that causes a rendering of an alert to an operator, which may be with a visualization or a dashboard.

In various embodiments, the temperature and/or concentration of gas(es) at one or more gas sources 110 may be controlled by the system 100, such as by causing the gas source to be cooled and or causing air to be allowed into the space. Once the system 100 receives a gas sample at the gas analyzer unit 150, the gas monitors 410 measures gas concentration and determine that the concentration is above one or more threshold in a gas source or that a temperature is greater than a threshold for concentration of gas in the gas source 110. The threshold has been determined by the gas analyzer unit 150 based on a calibration and prior historical measurement data and data from a battery control system 164. On the determination that the concentration is above a threshold, the system 100 may cause a cooling system to operate. The cooling system may supply the gas source 110 with forced cooling or may cause air to be provided to or extracted to the gas source 100 (e.g., opening one or more louvers).

Having generally described embodiments of a system 100 in accordance with the present invention, several exemplary operations according to exemplary embodiments will be described.

### Exemplary Operations

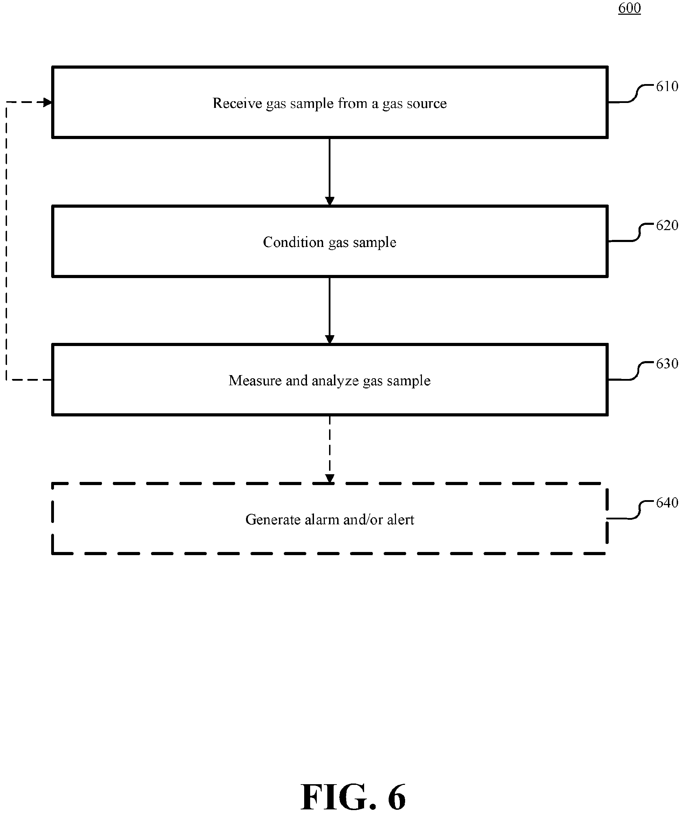

In some example embodiments, and according to the operations described herein, the system 100 may measure and analyze gases and air quality from multiple points. FIGS. 6-9 illustrate flowcharts associated with these operations. While the following description includes multiple operations, it is readily appreciated that some of the following operations omitted and that additional operations may be included. As is also readily appreciated, some of these operations may be repeated. Additionally, the order of operations should not be interpreted as limiting as the order of these operations may be varied. The operations described herein may be executed by the system 100, including by a processor, such as a processor 501 executing instructions stored in memory 503.

FIG. 6 illustrates a flowchart according to an example method for measurement and analysis of multipoint gases and air quality in accordance with one or more embodiments of the present disclosure.

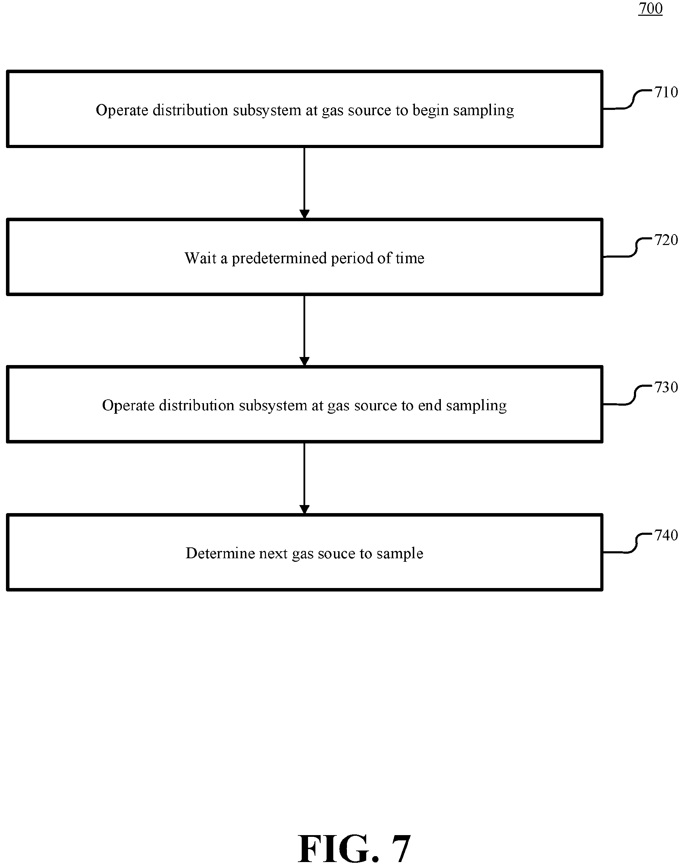

At operation 610, the system 100 receives a gas sample from a gas source 110. Operations associated with receiving a gas sample from a gas source 100, such as via a distribution subsystem 120, are further described in relation to FIG. 7.

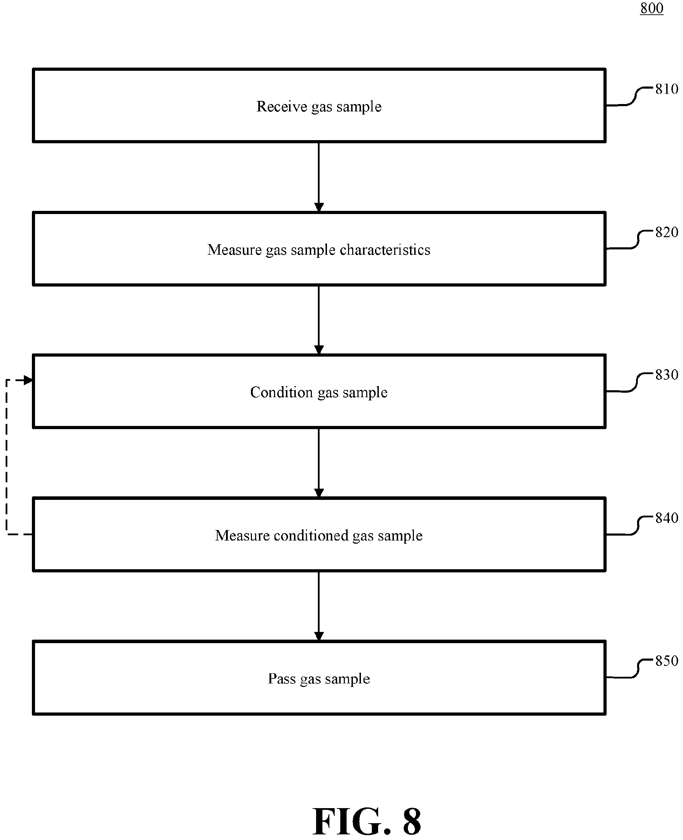

At operation 620, the system 100 conditions the gas sample. Operations for conditioning a gas sample from a gas source 110, such as with a gas conditioning subsystem 140, are further described in relation to FIG. 8.

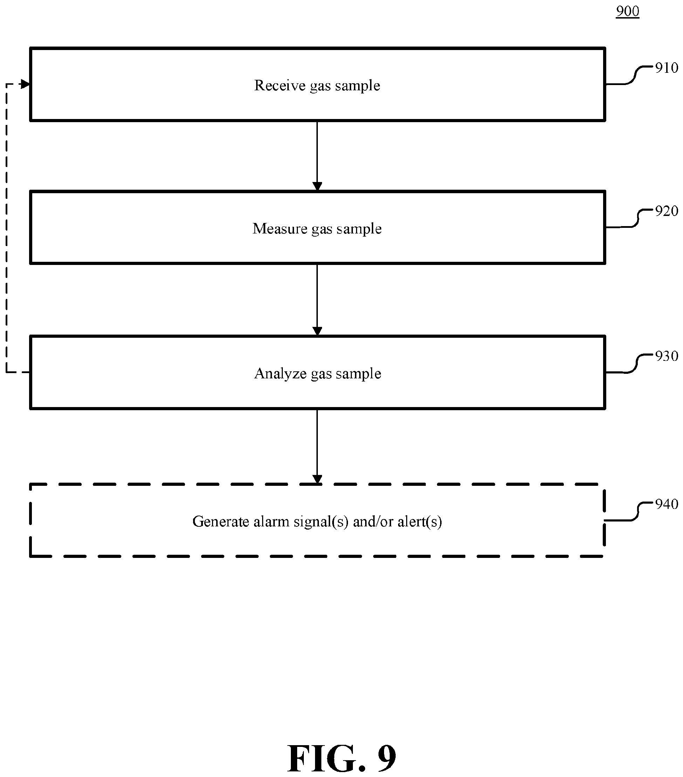

At operation 630, the system 100 measures and analyzes the gas sample. Operations for measuring and analyzing a gas sample are further described in relation to FIG. 9.

If the measurements and analysis of a gas sample does not lead to an alarm state, the system 100 may go back to operation 610 to receive a gas sample from another of the multiple gas sources 110.

At operation 640, the system 100 generates an alarm and/or alert. The generation of an alarm may include generating and transmitting one or more alarm signals, such as with input/output circuitry 507 and/or communications circuitry 505. In various embodiments, an alarm signal may be a binary signal, which may indicate there is an alarm or there is not an alarm. In various embodiments, the system 100 may be communicatively coupled to a control panel 164. The communicatively coupling may include an analog signal, a digital signal, and/or a network connection, which may be redundant connections. The generation of an alarm signal may include generating an analog signal or a digital signal to a predetermined value to indicate an alarm (e.g., change a 4-20 mA signal, change a 0 to a 1, etc.) and transmitting that alarm signal over the connection. A network connection may allow for data to be transmitted to the control panel 164, which may include the system 100 generating a data object containing data associated with the generation of the alarm signal. In various embodiments, the data object may include the current measurements (e.g., gas detected, concentration, temperature, etc.) and historical gas measurements along with environmental data associated with the generation of the alarm. The system 100 may also generate an alert, which may include a light, a display, a noise, and/or the composition of an electronic message (e.g., text string, visualization, email, text message, data push, etc.) and transmission of the electronic message to one or more computing devices associated with one or more users.

FIG. 7 illustrates a flowchart according to an example method for operating a distribution subsystem in accordance with one or more embodiments of the present disclosure.

At operation 710, the system 100 operates a distribution subsystem at a gas source to begin sampling. The distribution subsystem 120 may include one or more valves, which may be operated one at a time such that a gas sample may be taken from a gas source 110A while no gas samples are taken from other gas sources 110B, 110N. An open signal may be generated by the gas analyzer unit 150 and transmitted to a valve 122A to cause the valve 122 to open. In various embodiments a valve 122 may stay open as long as a signal is applied. Alternatively a valve 122 may open on a first signal and close on a second signal. Gas samples may be collected in parallel in the gas distribution system 120 but received sequentially by a gas monitor 410 in a gas analyzer unit 150 by operation of one or more valves (e.g., 122A, 122B, 122N) associated with the distribution subsystem 120, aspiration subsystem 130, gas conditioning subsystem 140, and/or gas analyzer unit 150 or by operation (or lack thereof) by one or more of the aspiration pumps 132.

At operation 720, the system 100 waits a predetermined period of time. The system 100 may control the size of a gas sample by controlling the vacuum and suction generated by an aspiration pump 132. By controlling the suction as well as a period of time that a value 122 is open, the system 100 may generate a consistent, estimated, or known gas sample size. The system 100 may also vary the gas sample size, which may be associated with a faster cycle time for receiving samples from each of the gas sources 110.

Additionally, the period of time may be varied from gas source 110A to gas source 100B in association with a determined or estimated time for a gas source to generate a concentration of gas. For example, if the system 100 measures a first concentration of a first gas from a gas source 100A that the system 100 associates with a potential thermal runaway of one or more batteries at the gas source 110, the system 100 may determine that a second gas sample of a specific size would have a second concentration that is associated with thermal runaway at a second time. Thus the system 100 may cause the valve 122 to open for a short period of time to generate a gas sample from the gas source 110 in order to take a subsequent measurement and determine if the measurement is at or above a threshold indicative of thermal runaway at this second period of time. These periods of the time and thresholds may be different, which may be determined according to an preset algorithm loaded during calibration or may be determined based on a machine learning model.

At operation 730, the system 100 operates a distribution subsystem at a gas source to end sampling. After the predetermine period of time has elapsed, the system 100 operates the valve 122 to close to complete the collection of the gas sample.

At operation 740, the system 100 determines the next gas source to sample. After a first gas sample has been received from a first gas source 110A, the system 100 may determine the next gas source (e.g., 110A, 110B, or 110N) to receive a gas sample from. The system 100 may take a second gas sample from the same gas source 110A or may take a gas sample from a different gas source 110B or 110N. In various embodiments, the gas sources may be sampled in sequential order. Alternatively, or additionally, the system 100 may sample the gas sources 110 in an order until a first threshold is exceeded by a gas sample associated with a first gas source 110 and then, the system 100 may take another gas sample from the first gas source 110 or may wait a predetermined period of time to take a second gas sample from the first gas source 110, which may cause the system 100 to take a second gas sample from the first gas source 110 out of order.

FIG. 8 illustrates a flowchart according to an example method for operating a gas conditioning subsystem in accordance with one or more embodiments of the present disclosure.

At operation 810, the gas conditioning subsystem 140 receives a gas sample. The gas conditioning system may be comprised of multiple gas conditioners 142A, 142B, 142N or it may be comprised of one gas conditioner 142. In various embodiments, each gas conditioner may include one or more valves to control receiving a gas sample as well as passing a gas sample. The system 100, such a via the gas conditioning subsystem 140, may operation an inlet valve to open to receive a gas sample into a gas conditioner 142.

At operation 820, the gas conditioning subsystem 140 measures the gas sample's characteristics. The gas conditioning subsystem 140 and/or the gas conditioner 142 may include one or more measurement devices to measure one or more characteristics (e.g., temperature, pressure, humidity, etc.) of a gas sample. A measurement device may include a temperature monitor (e.g., thermometer) to measure the temperature of a gas sample, a humidity monitor to measure the humidity of a gas sample, etc. If the measurement of the characteristic is above or below a threshold then the gas sample may be conditioned to bring the characteristic to below or above a threshold or within a range.

At operation 830, the gas conditioning subsystem 140 conditions the gas sample. The gas sample, having determined one or more characteristics of a gas sample did not meet a threshold or are outside of a range, may condition the gas sample, such as with gas conditioner 142. The conditioning may occur via a process (e.g., passing the gas sample through a condenser or radiator) or may occur for a period of time (e.g., multiple passes through a condenser or radiator).

At operation 840, the gas conditioning subsystem 140 measures the conditioned gas sample. After conditioning the gas sample, the gas conditioner subsystem may take measurements of the gas sample to confirm that the gas sample has passed a characteristic threshold or is within a range. If the gas sample has not passed, the conditioner may iterate operation 830 or may extend the time a gas sample is exposed to operation 830.

At operation 850, the gas conditioning subsystem 140 passes the gas sample. The system 100, such as with the gas conditioner subsystem 140, may determine that the measurements of the gas sample characteristics are below or above a threshold or within a range, and the system 100 may pass the gas sample to the gas analyzer unit 150. Passing the gas sample may include operating one or more valves, such as opening the valves, to cause the gas sample to pass to the gas analyzer unit 150.

FIG. 9 illustrates a flowchart according to an example method for measuring and analyzing gas samples in accordance with one or more embodiments of the present disclosure.

At operation 910, the gas analyzing unit 150 receives a gas sample. The gas analyzer unit 150 may include one or more gas monitors 410 as well as one or more valves to distribute a gas sample to the one or more gas monitor 410 within the gas analyzer unit 150. The distribution of the gas sample may be associated with the gas source, including distributing a first gas sample from a first gas source 110A to a first gas analyzer 410A and distributing a second gas sample from a second gas source 110B with a second gas analyzer 410B. In various embodiments, each gas monitor 410 may be associate with one or more valves to control receiving a gas sample to the gas monitor 410 as well as one or more valves to pass the gas sample to an exhaust 420.

At operation 920, the gas analyzing unit 150 measures the gas sample. After the gas sample is received at the gas monitor 410, the gas monitor 410 may measure one or more characteristics of the gas sample. The characteristics measured may include, but is not limited to, identification of gas(es), concentration of gas(es), temperature, humidity, air quality, etc. As the gas monitor 410 is generating measurements, the gas monitor 410 may provide the gas sample measurements to a computing device 430 of the gas analyzer unit 150 to analyze the gas sample measurements. The gas sample measurements may be provided in one or more data objects, which may include one or more data fields for each characteristics measured. Additionally, the data objects may include metadata, including but not limited to, time stamps, gas monitor identifier, etc. In various embodiments, the gas analysis described herein may be performed by the gas monitor 410A.

At operation 930, the gas analyzing unit 150 analyzes the gas sample. A computing device 430, such as in the gas analyzer unit 150 or in a gas monitor 410, may analyze the gas sample measurements. The analysis may occur in real time. The analysis may compare current gas sample measurements to one or more thresholds and or more ranges to determine if there is an alarm state. For example, if a gas concentration associated with off gassing of batteries is above a threshold for a first gas then an alarm state may be determined associated with a battery condition that may be indicative of thermal runaway.

In various embodiments, the analysis may include analyzing current gas measurement samples with historic gas measurement samples, which may be stored in one or more data objects in memory 503. For example, a comparison of current gas sample measurements with historic gas sample measurements may determine a rate of change in gas concentration and a rate of change with temperature that is indicative of a thermal runaway event. The comparison may be determinative that a thermal runaway event is occurring and there is an alarm state. Alternatively, the analysis may determine that based on the current gas sample measurement and the historical gas sample measurements a thermal runaway event is likely or has occurred and that a subsequent gas sample is to be taken from the gas source 110. The system 100 may generate signals to control one or more subsystems to collect a same from the gas source 110 for measurement and analysis.

Various embodiments include analyzing the current gas sample measurements with historical gas sample measurements from the current gas source 110A and other gas sources 110B, 110N. The system 100 may include a model or algorithm that associates how variations in a current gas sample from a current gas source 110A should be above or below a threshold or within a range based on historic gas sample measurements associated with the current gas source 110A as well as other gas sources 110B, 110N. The analysis may include a rate of change, a direction of change, an amount of change, and a time period, which may be indicative of an alarm state, such as hazardous environment being generated.

In an exemplary embodiment, a first gas source 110A may be associated with a first process step in a clean room and a second gas source 110B may be associated with a second process step. The current gas source may be the second gas source 110B, and the current gas sample measurements may be based on a gas sample measurement from the first gas source 110A, which would be indicative of a first process step occurring. The first process step may be the application of a chemical during the manufacturing of a semiconductor. After the first process step occurs, the manufacturing process may move the semiconductor being processed from a first station associated with the first gas source 110A to a second station associated with the second gas source 110B. A deviation in the manufacturing process at the second station associated with the current, second gas source 110B may result in a current gas sample measurement 110B that is above (or below) a threshold or outside a range. This deviation may, for example, be the result of excess heat, a fire, or an unintended chemical process. The analysis of the current gas sample from gas source 110B may be based on historic gas sample measurements associated with gas sources 110A and 110B, which may determine that there is an alarm state indicative of, for example, a fire or unintended chemical reaction at the second manufacturing station but not the first manufacturing station.

Additionally, after detecting an alarm state, the system 100 may determine to quickly cycle through each of the gas sources 110 to determine if the spaces associated with each of the gas sources 110 may need to be associated with an alarm. The system 100 may control the taking of gas samples from each of the gas sources 110, which may be at shorter times than when there is no alarm in order to expeditious cycle through the gas sources 110. If analysis of such additional gas samples in view of the historical gas samples associated with the alarm state, which may be from other gas sources, generates further alarm states then the system 100 may generate alarms and alerts as appropriate from each of the gas sources 110.

In various embodiments, the current gas sample measurements and historical gas sample measurements may be used to analyze inferences for one or more characteristics that may not be directly measured. Such inference may include, but are not limited to, concentrations, flow rates, or compositions of gas samples, such as future gas samples, or of events occurring in a space associated with a gas source.

In various embodiments, a first gas source 110A may be associated with a gas inlet of a space and a second gas source 110B may be associated with a gas outlet of space. If system 100 historic gas sample measurements from gas source 110A of the inlet and current gas sample measurements from gas source 100B of the outlet are different, then an inference may be determined about the gas concentration inside of the space, which may be a confined space or a hazardous space. Additionally, by using historical gas sample measurements of each gas source 110A, 110B and averaging the gas concentrations, an inferred gas concentration may be determined. The inference may be associated with an alarm state that causes the system 100 to generate an alarm or an alert.

The analysis may additionally include data from one or more sources outside of the system 100, such as from a computing device 162 or a control panel 164. In an exemplary embodiment with a battery control panel 164, the battery control panel 164 may provide one or more data regarding the state of the battery (e.g., charging, discharging, etc.), temperature, current voltage, maximum voltage of last full charge, time to discharge, etc. The system 100 may have one or more models or algorithms associated with the gas source 110 that associated one or more gas concentrations with an alarm state when a battery is, for example, charging and maximum voltage, which may be different from a model or algorithm associated with when a battery is discharging. For example, if a maximum voltage for a current charge is 98% of a maximum charge possible, indicating a 2% lower voltage than maximum charge, this may infer that the batteries have degraded over time and, thus, gas sample measurements may be associated with a different model or algorithm or different bias or starting point in using a model or algorithm. In such an embodiment, the data from the battery control panel 164 may be used in the analysis, including the selection of a model or algorithm, of a threshold, and/or of a range. In alternative embodiments, an equipment control panel 164 may provide if an equipment is operating or not operating, which may change the acceptable characteristics of a gas sample measurement.

For example, the equipment may be a coal pulverizer which may generate a large amount of coal particles in the air. The gas analyzer unit 150 may use different thresholds and or ranges depending on if this equipment is operating or not operating.

At operation 940, the gas analyzing unit 150 generates alarm signal(s) and/or alert(s). The alarm may include an alarm signal that may cause the operation of a purging subsystem 170 or a cooling subsystem to operate as described herein. The alert may include an alert to a computing device 162 that causes a rendering of an alert to an operator, which may be with a visualization or a dashboard as described herein.

In accordance with the invention, the gas sample measurements are used to determine an alarm state based on machine learning models, which may be used generate one or more threshold and/or ranges associated with an alarm state.

The system 100, such as with computing device 430, stores historic gas sampling measurements in data objects as well as data received from external sources, such as computing device 162 and/or control panels 164. Such data as well as historical alarm states may be used as machine learning training data sets. A machine learning model is trained with the machine learning training data sets to generate and output a model and/or algorithm that may be used by the system 100 to determine one or more alarm states based on a current gas sample measurement. The outputted model and/or algorithm may be in one or more data objects, which may be received or transmitted by the system 100. A model or an algorithm may also include scenarios or decisions trees that may relate how multiple gas sample measurements over time may be associated with an alarm state.

A machine learning model may be continuously trained and updated as new gas sample measurements are generated. In various embodiments, which may require noting disturbing the functioning of the system 100, the machine model may be updated but the updates may not be loaded to the system 100 while it is operating. Instead, the system 100 may be updated during a calibration period, which may occur based on an input received from a user, such as via input/output circuitry 507, computing device 160, or a control panel 164, which may be received at the same time as an calibration update.

In various embodiments, the machine learning may occur in the system 100 or remotely from the system 100, such as at computing device 162, and the system 100 may receive an update which may contain the models, algorithms, thresholds, and/or ranges generated or revised by the machine learning model. The computing device 162 may provide such an update in one or more data objects transmitted to the system 100, and the system 100 may be updated based on these data objects.

Operations of the present invention have been described in flowcharts. As will be appreciated, computer program instructions may be loaded onto a computer or other programmable apparatus (e.g., hardware) to produce a machine, such that the resulting computer or other programmable apparatus implements the functions and/or operations described in the flowchart blocks herein. These computer program instructions may also be stored in a computer-readable memory that may direct a computer or other programmable apparatus to function in a particular manner, such that the instructions stored in the computer-readable memory produce an article of manufacture, the execution of which implements the functions and/or operations described in the flowchart blocks. The computer program instructions may also be loaded onto a computer or other programmable apparatus to cause a series of operations to be performed on the computer or other programmable apparatus to produce a computer-implemented process such that the instructions executed on the computer or other programmable apparatus provide operations for implementing the functions and/or operations specified in the flowchart blocks. The flowchart blocks support combinations of means for performing the specified functions and combinations of operations for performing the specified functions and/or operations. It will be understood that one or more blocks of the flowcharts, and combinations of blocks in the flowcharts, can be implemented by special purpose hardware-based computer systems which perform the specified functions and/or operations, or combinations of special purpose hardware with computer instructions.

While this specification contains many specific embodiments and implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

While operations are illustrated in the drawings in a particular order, this should not be understood as requiring that such operations be performed in the particular order shown or in sequential order, or that all illustrated operations be performed, to achieve desirable results. In certain circumstances, operations in alternative ordering may be advantageous. In some cases, the actions recited in the claims may be performed in a different order and still achieve desirable results. Thus, while particular embodiments of the subject matter have been described, other embodiments are within the scope of the following claims.

## Claims

1. An aspiration based system comprising:
a distribution subsystem (120) including an inlet for each of a plurality of gas sources (110A, 110B, 110N) each associated with a plurality of battery cells located in multiple spaces;
an aspiration subsystem (130) coupled to the distribution subsystem (120), wherein the aspiration subsystem (130) generates a vacuum;
a gas analyzing unit (150);
wherein, in operation, the aspiration based system is configured to:
operate the distribution subsystem (120) to collect, via the vacuum, a gas sample from each of the plurality of gas sources (110A, 110B, 110N) in a sequence via the associated inlet;
measure, with the gas analyzing unit (150), the gas samples collected to generate one or more gas sample measurements; determine, based on an alarm threshold, an alarm state for one or more of the gas sample measurements, **characterised in that** the alarm threshold is determined using a machine learning model trained based on historic gas sampling measurement data and data from a battery control system (164), wherein the data from the battery control system (164) is communicated to the gas analyzing unit (150),
wherein the data from the battery control system (164) comprises one or more measurements of a plurality of battery cells; and
generate an alarm based on the determination of the alarm state.

2. The aspiration based system of claim 1 further comprising a gas conditioning subsystem (140), wherein the gas conditioning subsystem (140) is connected to an output of the aspiration subsystem and an input to the gas analyzing unit (150); and
wherein, in operation, the aspiration based system is further configured to:
condition, prior to determining an alarm state, the gas samples collected.

3. The aspiration based system of claim 2, wherein the gas conditioning subsystem (140) comprises a plurality of gas conditioners.

4. The aspiration based system of claim 1 further comprising a purging subsystem (170).

5. The aspiration based system of claim 1 further comprising a cooling subsystem.

6. The aspiration based system of claim 1, wherein, in operation, the aspiration based system is further configured to:
generate an alert based on the determination of the alarm state; and
transmit the alert to a computing device (162) associated with a user.

7. A method to measure and analyze multiple gas sources comprising:
providing an aspiration based system comprising:
a distribution subsystem including an inlet for each of the plurality of gas sources each associated with a plurality of battery cells located in multiple spaces;
an aspiration subsystem coupled to the distribution subsystem, wherein the aspiration based subsystem generates a vacuum;
a gas analyzing unit;
operating the distribution subsystem to collect, via the vacuum, a gas sample from each of the plurality of gas sources in a sequence via the associated inlet;
measuring, with the gas analyzing unit, the gas samples collected to generate one or more gas sample measurements;
determining, based on an alarm threshold, an alarm state for one or more of the gas sample measurements, **characterised in that** the alarm threshold is determined using a machine learning model trained based on historical gas sampling measurement data and data from a battery control system (164), wherein the data from the battery control system (164) is communicated to the gas analyzing unit (150), wherein the data from the battery control system (164) comprises one or more measurements of a plurality of battery cells; and
generating an alarm based on the determination of the alarm state.

8. The method of claim 7, wherein the aspiration based system further comprises a gas conditioning subsystem, wherein the gas conditioning subsystem is connected to an output of the aspiration subsystem and an input to the gas analyzing unit; and
conditioning, prior to determining an alarm state, the gas samples collected.

9. The method of claim 7, wherein the aspiration based system further comprises a purging subsystem.

10. The method of claim 7, wherein the aspiration based system further comprises a cooling subsystem.

11. The method of claim 7. further comprises:
generating an alert based on the determination of the alarm state; and
transmitting the alert to a computing device associated with a user.

12. The method of claim 7, wherein one or more of the plurality of gas sources are each associated with a plurality of battery cells.

## Patentansprüche

1. Ansaugbasiertes System, umfassend:
ein Verteilungsuntersystem (120), das einen Einlass für jede einer Vielzahl von Gasquellen (110A, 110B, 110N) beinhaltet, von denen jede einer Vielzahl von Batteriezellen zugehörig ist, die in mehreren Aussparungen angeordnet sind;
ein Ansauguntersystem (130), die mit dem Verteilungsuntersystem (120) gekoppelt sind, wobei das Ansauguntersystem (130) ein Vakuum erzeugt;
eine Gasanalyseeinheit (150);
wobei das ansaugbasierte System in Betrieb dazu konfiguriert ist:
das Verteilungsuntersystem (120) dazu zu betreiben, über das Vakuum eine Gasprobe von jeder der Vielzahl von Gasquellen (110A, 110B, 110N) in einer Reihenfolge über den zugehörigen Einlass zu erfassen;
mit der Gasanalyseeinheit (150) die erfassten Gasproben zu messen, um eine oder mehrere Gasprobenmessungen zu erzeugen; auf Basis eines Alarmschwellenwertes einen Alarmzustand für eine oder mehrere der Gasprobenmessungen zu bestimmen, **gekennzeichnet dadurch, dass** der Alarmschwellenwert unter Verwendung eines Maschinenlernen-Modells bestimmt wird, das auf Basis von historischen Gasprobenmessdaten und Daten aus einem Batteriesteuersystem (164) trainiert wird, wobei die Daten von dem Batteriesteuersystem (164) an die Gasanalyseeinheit (150) kommuniziert werden, wobei die Daten von dem Batteriesteuersystem (164) eine oder mehrere Messungen einer Vielzahl von Batteriezellen umfassen; und
einen Alarm auf Basis der Bestimmung des Alarmzustands zu erzeugen.

2. Ansaugbasiertes System nach Anspruch 1, ferner umfassend ein Gaskonditionierungsuntersystem (140), wobei das Gaskonditionierungsuntersystem (140) mit einem Ausgang des Ansauguntersystems und einem Eingang in die Gasanalyseeinheit (150) verbunden ist; und
wobei das ansaugbasierte System in Betrieb ferner dazu konfiguriert ist:
vor dem Bestimmen eines Alarmzustands die erfassten Gasproben zu konditionieren.

3. Ansaugbasiertes System nach Anspruch 2, wobei das Gaskonditionierungsuntersystem (140) eine Vielzahl von Gaskonditionierern umfasst.

4. Ansaugbasiertes System nach Anspruch 1, ferner umfassend ein Spüluntersystem (170).

5. Ansaugbasiertes System nach Anspruch 1, ferner umfassend ein Kühluntersystem.

6. Ansaugbasierte System nach Anspruch 1, wobei das ansaugbasierte System in Betrieb ferner dazu konfiguriert ist:
eine Warnung auf Basis der Bestimmung des Alarmzustands zu erzeugen;
die Warnung an eine Rechenvorrichtung (162), die einem Benutzer zugehörig ist, zu erzeugen.

7. Verfahren, um mehrere Gasquellen zu messen und zu analysieren, umfassend:
Bereitstellen eines ansaugbasierten Systems, umfassend:
ein Verteilungsuntersystem, das einen Einlass für jede der Vielzahl von Gasquellen beinhaltet, von denen jede einer Vielzahl von Batteriezellen zugehörig ist, die in mehreren Aussparungen angeordnet sind;
ein Ansauguntersystem, das mit dem Verteilungsuntersystem gekoppelt ist, wobei das ansaugbasierte Untersystem ein Vakuum erzeugt;
eine Gasanalyseeinheit;
Betreiben des Verteilungsuntersystems dazu, über das Vakuum eine Gasprobe von jeder der Vielzahl von Gasquellen in einer Reihenfolge über den zugehörigen Einlass zu erfassen;
Messen, mit der Gasanalyseeinheit, der erfassten Gasproben, um eine oder mehrere Gasprobenmessungen zu erzeugen;
Bestimmen, auf Basis eines Alarmschwellenwertes, eines Alarmzustands für eine oder mehrere der Gasprobenmessungen, **gekennzeichnet dadurch, dass**
der Alarmschwellenwert unter Verwendung eines Maschinenlernen-Modells bestimmt wird, das auf Basis von historischen Gasprobenmessdaten und Daten aus einem Batteriesteuersystem (164) trainiert wird, wobei die Daten von dem Batteriesteuersystem (164) an die Gasanalyseeinheit (150) kommuniziert werden, wobei die Daten von dem Batteriesteuersystem (164) eine oder mehrere Messungen einer Vielzahl von Batteriezellen umfassen; und
Erzeugen eines Alarms auf Basis der Bestimmung des Alarmzustands.

8. Verfahren nach Anspruch 7, wobei das ansaugbasierte System ferner ein Gaskonditionierungsuntersystem umfasst, wobei das Gaskonditionierungsuntersystem mit einem Ausgang des Ansauguntersystems und einem Eingang in die Gasanalyseeinheit verbunden ist; und
Konditionieren, vor dem Bestimmen eines Alarmzustands, der erfassten Gasprobe.

9. Verfahren nach Anspruch 7, wobei das ansaugbasierte System ferner ein Spülsystem umfasst.

10. Verfahren nach Anspruch 7, wobei das ansaugbasierte System ferner ein Kühlsystem umfasst.

11. Verfahren nach Anspruch 7, ferner umfassend:
Erzeugen einer Warnung auf Basis der Bestimmung des Alarmzustands; und
Senden der Warnung an eine Rechenvorrichtung, die einem Benutzer zugehörig ist.

12. Verfahren nach Anspruch 7, wobei eine oder mehrere der Vielzahl von Gasquellen jeweils einer Vielzahl von Batteriezellen zugehörig sind.

## Revendications

1. Système basé sur l'aspiration comprenant :
un sous-système de distribution (120) incluant une entrée pour chacune d'une pluralité de sources de gaz (110A, 110B, 110N), chacune associée à une pluralité de cellules de batterie situées dans de multiples espaces ;
un sous-système d'aspiration (130) couplé au sous-système de distribution (120), dans lequel le sous-système d'aspiration (130) génère un vide ;
une unité d'analyse de gaz (150) ;
dans lequel, en fonctionnement, le système basé sur l'aspiration est configuré pour :
faire fonctionner le sous-système de distribution (120) pour collecter, via le vide, un échantillon de gaz provenant de chacune de la pluralité de sources de gaz (110A, 110B, 110N) dans une séquence via l'entrée associée ;
mesurer, avec l'unité d'analyse de gaz (150), les échantillons de gaz collectés pour générer une ou plusieurs mesures d'échantillon de gaz ; déterminer, sur la base d'un seuil d'alarme, un état d'alarme pour une ou plusieurs des mesures d'échantillon de gaz, **caractérisé en ce que** le seuil d'alarme est déterminé en utilisant un modèle d'apprentissage automatique entraîné sur la base de données historiques de mesure d'échantillonnage de gaz et de données provenant d'un système de contrôle de batterie (164), dans lequel les données provenant du système de contrôle de batterie (164) sont communiquées à l'unité d'analyse de gaz (150), dans lequel les données provenant du système de contrôle de batterie (164) comprennent une ou plusieurs mesures d'une pluralité de cellules de batterie ; et
générer une alarme sur la base de la détermination de l'état d'alarme.

2. Système basé sur l'aspiration selon la revendication 1 comprenant en outre un sous-système de conditionnement de gaz (140), dans lequel le sous-système de conditionnement de gaz (140) est relié à une sortie du sous-système d'aspiration et à une entrée de l'unité d'analyse de gaz (150) ; et
dans lequel, en fonctionnement, le système basé sur l'aspiration est en outre configuré pour :
conditionner, avant la détermination d'un état d'alarme, les échantillons de gaz collectés.

3. Système basé sur l'aspiration selon la revendication 2, dans lequel le sous-système de conditionnement de gaz (140) comprend une pluralité de conditionneurs de gaz.

4. Système basé sur l'aspiration selon la revendication 1, comprenant en outre un sous-système de purge (170).

5. Système basé sur l'aspiration selon la revendication 1, comprenant en outre un sous-système de refroidissement.

6. Système basé sur l'aspiration selon la revendication 1, dans lequel, en fonctionnement, le système basé sur l'aspiration est en outre configuré pour :
générer une alerte sur la base de la détermination de l'état d'alarme ; et
transmettre l'alerte à un dispositif informatique (162) associé à un utilisateur.

7. Procédé pour mesurer et analyser de multiples sources de gaz comprenant :
la fourniture d'un système basé sur l'aspiration comprenant :
un sous-système de distribution incluant une entrée pour chacune de la pluralité de sources de gaz, chacune associée à une pluralité de cellules de batterie situées dans de multiples espaces ;
un sous-système d'aspiration couplé au sous-système de distribution, dans lequel le sous-système basé sur l'aspiration génère un vide ;
une unité d'analyse de gaz ;
le fonctionnement du sous-système de distribution pour collecter, via le vide, un échantillon de gaz provenant de chacune de la pluralité de sources de gaz dans une séquence via l'entrée associée ;
la mesure, avec l'unité d'analyse de gaz, des échantillons de gaz collectés pour générer une ou plusieurs mesures d'échantillon de gaz ;
la détermination, sur la base d'un seuil d'alarme, d'un état d'alarme pour une ou plusieurs des mesures d'échantillon de gaz, **caractérisé en ce que**
le seuil d'alarme est déterminé en utilisant un modèle d'apprentissage automatique entraîné sur la base de données historiques de mesure d'échantillonnage de gaz et de données provenant d'un système de contrôle de batterie (164), dans lequel les données provenant du système de contrôle de batterie (164) sont communiquées à l'unité d'analyse de gaz (150), dans lequel les données provenant du système de contrôle de batterie (164) comprennent une ou plusieurs mesures d'une pluralité de cellules de batterie ; et
la génération d'une alarme sur la base de la détermination de l'état d'alarme.

8. Procédé selon la revendication 7, dans lequel le système basé sur l'aspiration comprend en outre un sous-système de conditionnement de gaz, dans lequel le sous-système de conditionnement de gaz est relié à une sortie du sous-système d'aspiration et à une entrée de l'unité d'analyse de gaz ; et
le conditionnement, avant la détermination d'un état d'alarme, des échantillons de gaz collectés.

9. Procédé selon la revendication 7, dans lequel le système basé sur l'aspiration comprend en outre un sous-système de purge.

10. Procédé selon la revendication 7, dans lequel le système basé sur l'aspiration comprend en outre un sous-système de refroidissement.

11. Procédé selon la revendication 7, comprenant en outre :
la génération d'une alerte sur la base de la détermination de l'état d'alarme ; et
la transmission de l'alerte à un dispositif informatique associé à un utilisateur.

12. Procédé selon la revendication 7, dans lequel une ou plusieurs de la pluralité de sources de gaz sont chacune associées à une pluralité de cellules de batterie.
